# EUROPEAN PATENT SPECIFICATION

(11) **EP 1 561 105 B1**
(45) Date of publication and mention of the grant of the patent: **04.11.2009**
(21) Application number: 03785733.1
(22) Date of filing: 06.11.2003
(51) Int. Cl.: G01N 33/50, G01N 33/68

(54) **METHOD FOR PROVIDING NATURAL THERAPEUTIC AGENTS WITH HIGH THERAPEUTIC INDEX**
VERFAHREN ZUM BEREITSTELLEN VON NATÜRLICHEN ARZNEIMITTEL MIT HOHEN THERAPEUTISCHEN INDIZES
PROCEDE POUR FOURNIR DES AGENTS THERAPEUTIQUES NATURELS A INDICE THERAPEUTIQUE ELEVE

(30) Priority: 07.11.2002 EP 02292787; 10.12.2002 US 315493
(43) Date of publication of application: 10.08.2005
(73) Proprietor: GenOdyssee, 91058 Evry Cedex (FR)
(72) Inventor: ESCARY, Jean-Louis, F-78150 Le Chesnay (FR)
(74) Representative: Caen, Thierry Alain
(86) International application number: PCT/EP2003/013695
(87) International publication number: WO 2004/042394

(56) References cited:
- WO-A-02/079249
- WO-A-02/086156
- ORTALDO J R ET AL: "EFFECTS OF SEVERAL SPECIES OF HUMAN LEUKOCYTE INTERFERON ON CYTO TOXIC ACTIVITY OF NATURAL KILLER CELLS AND MONOCYTES" INTERNATIONAL JOURNAL OF CANCER, vol. 31, no. 3, 1983, pages 285-290, XP008016137 ISSN: 0020-7136
- PESTKA S: "The human interferons--from protein purification and sequence to cloning and expression in bacteria: before, between, and beyond." ARCHIVES OF BIOCHEMISTRY AND BIOPHYSICS. UNITED STATES 15 FEB 1983, vol. 221, no. 1, 15 February 1983 (1983-02-15), pages 1-37, XP008016138 ISSN: 0003-9861
- SPERBER S J ET AL: "ANTI-HIV-1 ACTIVITY OF RECOMBINANT AND HYBRID SPECIES OF INTERFERON-ALPHA" JOURNAL OF INTERFERON RESEARCH, MARY ANN LIEBERT, INC., NEW YORK, NY, US, vol. 12, 1992, pages 363-368, XP001038692 ISSN: 0197-8357
- EVINGER M ET AL: "ANTI PROLIFERATIVE AND ANTI VIRAL ACTIVITIES OF HUMAN LEUKOCYTE INTERFERONS" ARCHIVES OF BIOCHEMISTRY AND BIOPHYSICS, vol. 210, no. 1, 1981, pages 319-329, XP008016158 ISSN: 0003-9861
- TROWN P.W. ET AL CANCER vol. 57, no. SUPPL., 1986, pages 1648 - 1656

## Description

### FIELD OF THE INVENTION

The present invention relates to a method for providing therapeutic agents in particular polypeptides having a high therapeutic index, as well as polynucleotides encoding said polypeptides.

### BACKGROUND OF THE INVENTION

The aim of drug discovery is to develop safe and effective drug. Many methods are currently used in this purpose. Generally, after having identified and validated a target, a large number of molecules are screened against these targets to identify those molecules that have the potential to progress through the lengthy and expensive drug development process.

To develop new therapeutic agents from peptide and/or protein compounds, different approaches may be followed. One possible approach is to strategically design a library of peptide compounds by introducing random mutation on positions of the molecule that are liable to improve functionality. Alternatively, random mutations may be introduced throughout the gene using for example error-prone PCR or an *Escherichia coli* strain lacking DNA repair mechanisms. Another interesting approach consists of generating sequence diversity by DNA shuffling (Stemmer (1994), Nature 370, 389-391 and Coco et al. (2001), Nature Biotechnology 19, 354-359). This method consists in randomly amplifying and fragmenting related DNA sequences, then reassembling the fragments using DNA polymerase in a self-priming fashion. The resulting chimeric molecules are selected and can be bred again, accumulating multiple beneficial mutations. This method belongs to *in vitro* or *in vivo* recombination based techniques such as directed evolution technologies which are techniques well known to the one skilled in the art. In complement of these methods, computational approaches may also be suitable to increase rational protein design and reduce the number of molecules on which to perform the experimental tests (Hellinga (1998), Nat. Struct. Biol. 5:525-527; DeGrado et al. (1999), Annu. Rev. Biochem. 68:779-819; Gordon et al. (1999) Curr. Opin. Struct. Biol. 9: 509-513; Janin (1996) Proteins 25:438-445).

Each of the methods described above has its own advantages and limitations. As limitations, we can quote the necessity to evaluate a large number of molecules, the necessity to dispose of sufficient structural information on the gene or the product of said gene, the necessity to generate a high and often useless number of related gene sequences and the necessity for a rational lead optimisation. Furthermore, these methods generate non-naturally occurring proteins which are susceptible to induce unexpected side effects in the patients, for instance immunogenicity.

In the past years, natural genetic polymorphisms (which include natural allelic variants of a gene) and polypeptides encoded thereby were known to be useful mainly for:
- linkage analysis, evolutionary studies, forensics.
- establishing phylogenetic relationships between different species.
- generally determining approximate levels of DNA sequence diversity both within and between living individuals.
- analysing genome diversity.
- identifying disease-causing gene mutations, genetic marker development, and the like (diagnostic/prognosis of a disease or pathology).
- providing large numbers of low mutating genetic markers for use in gene mapping.
- using forensics applications such as DNA fingerprinting.
- targets for drug discovery and drug development.

The effects of ten species of human leukocyte interferons (IFNα1, IFNα2, IFNβ1, IFNβ2, IFNβ3, IFNγ1, IFNγ2, IFNγ3, IFNγ4, IFNγ5) on cytotoxic activitly of NK cells and monocytes were described in Ortaldo et al. (Intern. J. Cancer (1983) 31(3):285-290).

The review by Pestka et al. (Archives Biochemistry Biophysics (1983) 221(1):1-37) discloses the action, the protein purification, and sequence cloning of human interferons, which are described as being useful as therapeutic agents.

The anti-HlV1 activity of nine recombinant and hybrid species of interferons was described by Sperber et al. (J. Interferon Res (1992)12: 363-368).

The antiproliferative and antiviral activities of human leukocyte interferons were disclosed by Evinger et al. (Archives Biochemistry Biophysics (1981) 210(1):319-329).

Natural allelic variants of IFN alpha 17 and IFN alpha 21 and their therapeutic uses have been disclosed in WO 02 086156 and WO 02 079249, respectively.

The antiviral activity of Roferon®-A (IFN alpha 2a) is compared with that of other human interferon alpha subtypes in Trown et al. (Cancer 57 (1986) 1648-1656).

Until now, natural allelic variants of genes, which are found in a given population, have not been regarded by the one skilled in the art as a source of molecules for providing a significant number of therapeutic agents, in particular therapeutic agents having high therapeutic efficiency.

Indeed, it could not be expected that polypeptides encoded by natural allelic variants of a same gene, differing by as little as only one amino acid, could have significantly different pharmacological properties and/or pharmacological profiles.

This was even less expected for proteins like cytokines which show pleiotropic activity.

It can be emphasized here that, if it might happen that one specific natural allelic variant was tested in a biological assay, it has not yet been proposed to use as many as possible of such variants to determine those which can actually be useful as therapeutic agents.

A method is needed which allows to simply select and provide, among several polypeptides encoded by natural allelic variants, those useful as therapeutic agents.

Therefore, one aspect of the present invention is a new method for providing new therapeutic agent(s), which overcomes some or all drawbacks of the above-mentioned known methods.

### BRIEF SUMMARY OF THE INVENTION

One aspect of the present invention is a method for identifying new therapeutic agent(s), characterized in that it comprises the following steps:
a) providing a reference product having a therapeutic index, said reference product being known to be useful in a least one therapeutic application and said therapeutic index corresponding to a balance between beneficial and adverse effects of said reference product;
b) selecting at least one polypeptide encoded by a natural allelic variant of one preselected gene with therapeutic potential belonging to the gene families of cytokines;
c) determining the therapeutic index of the polypeptide(s) selected in step b), said therapeutic index corresponding to a balance between beneficial and adverse effects of said polypeptide(s) selected in step b), said adverse effects not being determined through *in vivo* toxicity tests performed in humans;
d) retaining as therapeutic agent(s), the polypeptide(s) selected in step b) whose therapeutic index, as determined in step c), is higher than the therapeutic index of the reference product.

This method enables to identify, as therapeutic agents, polypeptides which were not known as such.

The natural allelic variants which encode the polypeptides selected in step a) can be natural allelic variants of any preselected gene with therapeutic potential, as defined hereunder, and/or of any gene that belongs to the same gene family as said preselected gene with therapeutic potential. In other words, the method of the present invention applies to polypeptides encoded by natural allelic variants of a known gene, of genes of the same gene family as said known gene, or of both a known gene and genes of the same gene family as said known gene.

The method of the invention may be applied to the natural allelic variants of one single gene that can be either a preselected gene with therapeutic potential or any gene belonging to the same gene family as said preselected gene.

Preferably, at least 2 polypeptides, and more preferably at least 4 polypeptides encoded by natural allelic variants of a preselected gene, and/or of any related gene, are selected in step a).

The maximum number of polypeptides that can be carried out in the method according to the present invention depends mainly on the number of identified natural allelic variants encoding such polypeptides.

When the preselected gene with therapeutic potential belongs to a gene family, the method of the invention may be preferably applied to natural allelic variants of several genes belonging to the same gene family as said preselected gene as well as to natural allelic variants of said preselected gene.

The natural allelic variants may originate from different species, but preferably originate from the same species, more preferably from the human species.

In another aspect of the invention, for each polypeptide obtainable according to the method of the invention, the therapeutic index is established by first submitting said polypeptides to at least two activity tests, second attributing a value in direct relation with the results of said activity tests, and finally determining the therapeutic index from the attributed values.

According to the invention, in step c), only the polypeptide(s) selected in step a) whose therapeutic index is higher than the therapeutic index of reference are retained as therapeutic agent(s).

In a preferred aspect of the invention, the polypeptides with the highest or second highest therapeutic index are selected as therapeutic agent(s). Thus, the preferred therapeutic agents of the invention are the polypeptides having the highest or second highest therapeutic index, it being understood that their therapeutic index is also higher than that of the reference product.

Also, the method of the invention may be used to provide new therapeutic agents with new pharmacological profiles, which may eventually become available for the development of new therapeutic applications.

Another aspect of the present invention concerns the use of the polypeptides provided by the method described herein, as well as the polynucleotides encoding said polypeptides, as well as their derivatives, as therapeutic agents for the manufacture of a medicament to be administered to a patient in need thereof.

### BRIEF DESCRIPTION OF THE DRAWINGS

Further understanding of the present invention may be had by reference to the figures wherein:

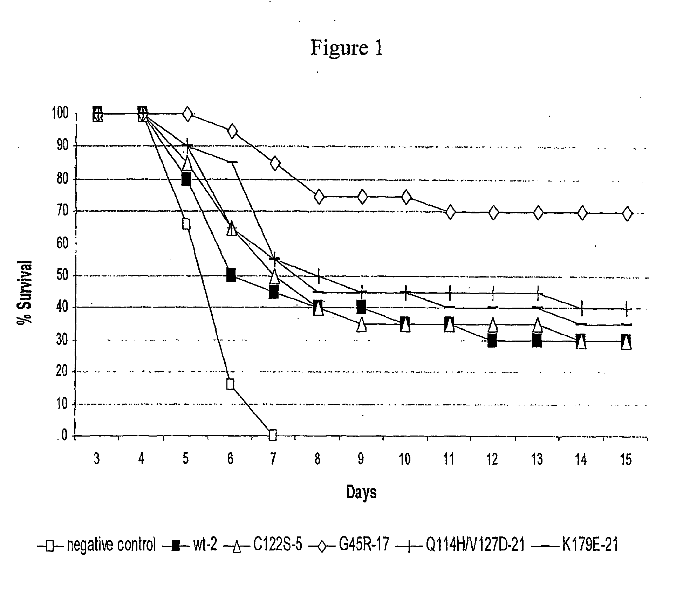

Figure 1 represents the survival rate of mice previously infected by EMCV virus and treated with one of five polypeptides encoded by natural allelic variants of genes belonging to the IFNα gene family, or those which received excipient only. In this figure, the abscissas correspond to the time of survival (days) and the ordinates correspond to the relative survival rate of EMCV infected mice. The interferons tested were: C122S IFNα-5 (Δ), G45R IFNα-17 (o), Q114H and V127D IFNα-21 (+), and K179E IFNα-21 (-), wild type IFNα-2 (v). A negative control (□) corresponding to excipient only is also presented.

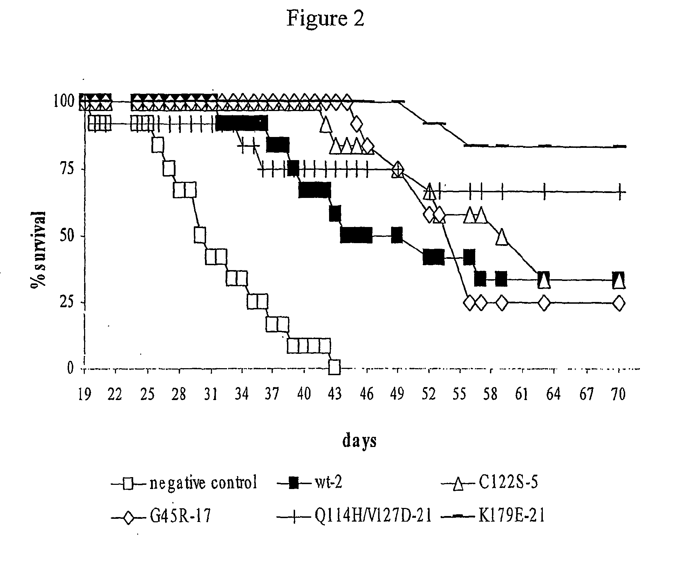

Figure 2 represents the survival rate of mice previously inoculated with malignant Friend erythroleukemia cells (FLC) and treated with one of five polypeptides encoded by natural allelic variants of genes belonging to the IFNα gene family, or those which received excipient only. In this figure, the abscissas correspond to the time of survival (days) and the ordinates correspond to the relative survival rate of FLC inoculated mice. The interferons tested were: C122S IFNα-5 (Δ), G45R IFNα-17 (◇), Q114H and V127D IFNα-21 (+), and K179E IFNα-21 (-), wild type IFNα-2 (v). A negative control (□) corresponding to excipient only is also presented.

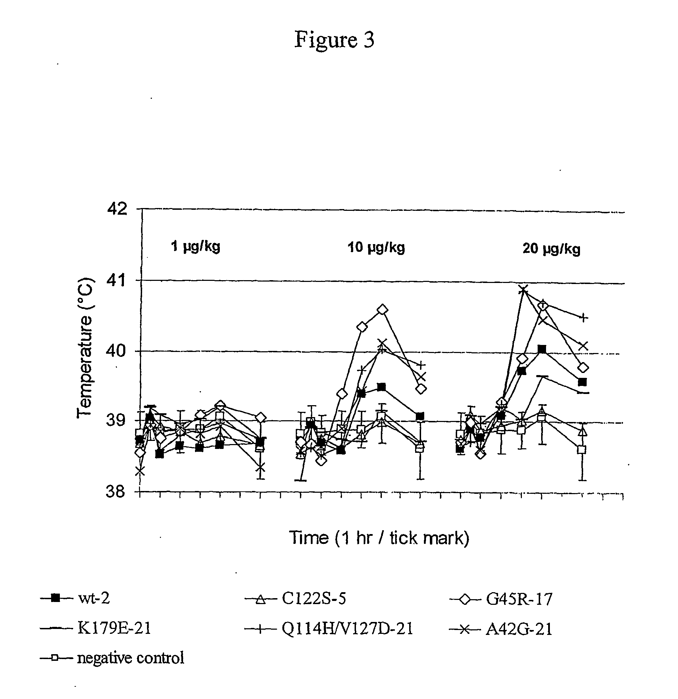

Figure 3 represents the effect of subcutaneous administration of one of five polypeptides encoded by natural allelic variants of genes belonging to the IFNα gene family on body temperature of Rhesus monkeys. Three doses of interferons were tested: 1, 10 or 20 µg/kg. In this figure, the abscissas correspond to the time after interferon administration (1 tip mark represents one hour) and the ordinates correspond to body temperature (°C). The interferons tested were: C122S IFNα-5 (Δ), G45R IFNα-17 (◇), Q114H and V127D IFNα-21 (+), and K179E IFNα-21 (-), wild type IFNα-2 (v). A negative control (□) corresponding to excipient only is also presented.

### DETAILED DESCRIPTION OF THE INVENTION

The following terms, used in the present description, have the meanings given below.

A "**therapeutic agent**" designates a substance that can be used in a medical treatment.

A "**preselected gene with therapeutic potential"** designates a known gene encoding at least one polypeptide, said polypeptide being selected from the group consisting of:
- polypeptides developed in the industry as therapeutic agents and which are already used on the market as therapeutic agents in at least one therapeutic application;
- polypeptides developed in the industry as therapeutic agents and which are not yet on the market, and/or;
- polypeptides which have a potential or assumed role in a metabolic or a biological pathway, which renders said polypeptides potentially useful as therapeutic agents. In order to determine if a polypeptide has a potential or assumed role in a metabolic or biological pathway, one may refer to biological, physiological, epidemiological, medical and clinical data made available to the experimenter with regard to the polypeptide or the nucleotide sequence encoding said polypeptide.

As an example, IFNα-2 is a polypeptide already used on the market, whereas IFNα-21 and IFNα-17, which belong to the same gene family, may be developed in the industry as therapeutic agents but are not available on the market yet.

The nucleotide sequence of said preselected gene with therapeutic potential is usually available in nucleotide sequence databases like those run by these official institutions: EMBL (European Molecular Biology Laboratory; Meyerhofstrasse 1 D-69117 Heidelberg, GERMANY), the NCBI (National Center for Biotechnology Information; National Library of Medicine Building 38A Bethesda, MD 20894, US) and DDBJ (DNA DataBase of Japan; 1111 Yata, Mishima, Shizuoka 411-8540, JAPAN).

A "**gene family**" comprises genes which:
- display enough nucleotide sequence identity;
- code for polypeptides with enough amino acid sequence homology; and/or
- share a common amino acid pattern which characterizes said family; to permit their classification as members of the same group.

The members of a gene family share the same or similar biological function(s) and have usually arisen from a common ancestor by gene duplication or amplification and have diverged subsequently by mutations.

The fact that a gene belongs to the same gene family as another gene is generally provided by the scientific literature, but also in the international nucleotide sequence databases such as those run by the official institutions previously mentioned. This information is usually further commented with respect to the gene's products in protein databases such as Swissprot^{®} that is available at SwissProt, EMBL Outstation - European Bioinformatics Institute, Wellcome Trust Genome Campus, Hinxton, Cambridge CB10 1SD, UK.

Alternatively, in particular if all the members of a gene family are not provided by the scientific literature, one skilled in the art may determine if a gene belongs to a gene family, and to which one, by running a sequence homology search in an appropriate nucleotide or protein database like the one quoted above using a tool such as "blast" (basic local alignment search tool), which is well known by those skilled in the art. The matches with the best score will correspond to members of said family. For example, one skilled in the art may also use the software based on this principle which performs such a homology search, and which is available on the internet site http://www.ebi.ac.uk/clustr.

A gene belonging to the same gene family as the preselected gene with therapeutic potential is not necessarily reported in the literature as having a therapeutic potential.

The following gene families are given as examples: the gene family encoding ribosomal RNA, the gene family encoding alpha-globins, the gene family encoding beta-globins, the gene family encoding human growth hormone, the gene family encoding actin proteins, the gene family encoding serine proteases, the gene family encoding vitellogenins, the gene family encoding the major histocompatibility antigens.

The following are examples of gene families encoding cytokines: the gene family encoding TGF-beta (T-cells Growth Factor), the gene family encoding EGF (Epithelial Growth Factors), the gene family encoding VEGF (Vascular Endothelial Growth Factor), the gene family encoding FGF (Fibroblast Growth Factors), the gene family encoding the chemokines, the gene family encoding the neurotrophins, the gene family encoding the IFNα (Interferons-alpha). One may note, for example, that the gene family encoding IFNα comprises at least 23 members like IFNα-2, IFNα-5, IFNα-17 and IFNα-21.

In the present invention, the term "**related gene**" applied to a preselected gene with therapeutic potential refers to any gene belonging to the same gene family, as defined above, as said preselected gene with therapeutic potential.

"**Polynucleotide**" is defined as a polyribonucleotide or a polydeoxribonucleotide that can be a modified or non-modified RNA or DNA.

The term polynucleotide includes, for example, single stranded or double stranded DNA, DNA comprising a mixture of one or several single stranded region(s) and of one or several double stranded region(s), single stranded or double stranded RNA, or RNA comprising a mixture of one or several single stranded region(s) and of one or several double stranded region(s). The term polynucleotide may also include RNA and/or DNA including one or several triple stranded regions and DNA and/or RNA containing one or several bases modified for reasons of stability or for other reasons. "Modified base" is understood to include, for example, the unusual bases such as inosine.

"**Natural allelic variants of a gene**" are defined, in the present invention, as polynucleotides present at the same locus as said gene, but differing in their nucleotide sequences as a result from non-synonymous coding genetic variations in the nucleotide sequence of said gene.

In the sense of the present invention, a natural allelic variant of a gene may result from a change in the nature of one or more nucleotides, a deletion, an insertion or a repetition of one or more nucleotides in the nucleotide sequence of said gene.

A non-synonymous coding genetic variation is a polymorphism in the coding sequence of a nucleotide sequence that involves a modification of at least one amino acid in the sequence of amino acids encoded by this nucleotide sequence. In this case, the genetic variation results in a variation in the amino acid sequence of the natural polypeptide.

"**Polypeptide**" is defined as a peptide, an oligopeptide, an oligomer or a protein comprising at least two amino acids joined to each other by a normal or modified peptide bond, such as in the cases of the isosteric peptides, for instance.

A polypeptide can be composed of amino acids other than the 20 amino acids defined by the genetic code. A polypeptide can equally be composed of amino acids modified by natural processes, such as post-translational maturation processes, which are well known to those skilled in the art. Such modifications are fully detailed in the literature. These modifications can appear anywhere in the polypeptide: in the peptide skeleton, in the amino acid chain or even at the carboxy- or amino-terminal ends.

A polypeptide can be branched following an ubiquitination or be cyclic with or without branching. This type of modification can be the result of natural post-translational processes that are well known to those skilled in the art. Such modifications are fully detailed in the literature: PROTEINS-STRUCTURE AND MOLECULAR PROPERTIES, 2nd Ed., T. E. Creighton, New York, 1993; POST-TRANSLATIONAL COVALENT MODIFICATION OF PROTEINS, B. C. Johnson, Ed., Academic Press, New York, 1983; Seifter et al. "Analysis for protein modifications and non protein cofactors", Meth. Enzymol. (1990) 182: 626-646; and Rattan et al. "Protein Synthesis: Post-translational Modifications and Aging", Ann NY Acad Sci (1992) 663: 48-62.

A polypeptide encoded by a natural allelic variant of a gene may have an increased, reduced or suppressed biological activity in comparison with the polypeptide encoded by another natural allelic variant of the same gene.

A polypeptide encoded by a natural allelic variant of a gene can equally have a biological activity whose nature is changed in comparison with that of the polypeptide encoded by another allelic variant of the same gene.

An "**activity test**" designates any experiment performed to evaluate a given biological or pharmacological activity, or a biochemical, biophysical and/or physico-chemical property of a potential therapeutic agent that may consist of a polypeptide. An activity test may reflect the beneficial activity sought in a given therapeutic application or an adverse effect, as for example, toxicity.

The activity test for a given therapeutic agent may, for example, enable one to determine the agent's proliferative or anti-proliferative (anti-tumoral) activity, its affinity or absence of affinity to a ligand and/or a receptor, its immunomodulatory effect, its antiviral activity, its effects on cell differentiation. It may also enable one to determine if the therapeutic agent may induce fever, immunogenicity or to evaluate the effect of the polypeptide on heart rate, blood pressure, appetite, hair loss, and depression. The activity test may also enable one to determine differences in the level of gene expression or in the half-life of a polypeptide. Physico-chemical properties may refer, for example, to solubility of a therapeutic agent into a medium, its compatibility with a given excipient, or its resistance to proteolysis. The literature, or personal knowledge, regarding the preselected gene will provide one skilled in the art with the different activity tests that can be performed and the standard protocols that are suitable to carry out the activity tests relevant in regard to a given therapeutic application.

A "**therapeutic index**" is a value that reflects the suitability of a therapeutic agent for a given therapeutic application. This value is generally a numerical value which may be arbitrarily attributed by an experimenter. This value is generally based on the results obtained from at least two activity tests, as defined above. For instance, the "therapeutic index" of a therapeutic agent corresponds to the balance between the benefits of said therapeutic agent for a given therapeutic application and the adverse effects of said therapeutic agent. The benefits of a therapeutic agent are related to its therapeutic action or efficacy that may be evaluated by *in vitro* and/or *in vivo* assays measuring the therapeutic agent's biological and pharmacological activity required for the intended therapeutic application. The adverse effects of a therapeutic agent may correspond to the toxicity evaluated *in vitro* and/or *in vivo* in different biological models.

Usually the therapeutic index of a polypeptide is determined with respect to a reference product, as defined below.

"**Therapeutic index of reference**" usually relates to the therapeutic index, as defined above, of a reference product. The reference product can be:
- a therapeutic agent already on the market;
- a therapeutic agent not yet on the market but developed or under development/testing in the industry in expectation of being marketed;
- any allelic variant of a preselected gene with therapeutic potential, as defined above.

Said reference product can be of variable nature such as a chemical compound, a natural or synthetic polypeptide, a polynucleotide, for example.

Usually, the reference product is chosen because it is known to be useful in a specific therapeutic application in which it is believed that the polypeptides to be retained as therapeutic agents by the method of the present invention may also be useful.

The determination of the therapeutic index of reference usually requires that the experimenter carries out, simultaneously or not, the same protocol or substantially the same protocol for the reference product and for the polypeptides that are to be compared.

As an example, interferon IFNα-2 can be a reference product with regard to polypeptides encoded by natural allelic variants of genes belonging to the interferon alpha gene family.

Alternatively, the therapeutic index of reference can be established from data available with respect to at least one reference product, whose data can be found in the scientific literature or from clinical data. In this case, for all the polypeptides that are investigated, the activity tests are usually performed according to the protocols provided by said scientific literature or said clinical data.

Therefore, the therapeutic index of reference may be determined independently of the therapeutic index of the polypeptides selected in step a) of the method of the invention.

The natural allelic variants of a preselected gene with therapeutic potential and/or of any related gene considered in the invention may originate from the locus of the preselected gene with therapeutic potential or from the locus of any related gene that belongs to the same gene family as said preselected gene with therapeutic potential.

According to the present invention the polypeptides selected in step a) are polypeptides encoded by natural allelic variants of one preselected gene with therapeutic potential and polypeptides encoded by natural allelic variants of at least one related gene, it being understood that the related gene is a gene - belonging to the same gene family as the preselected gene with therapeutic potential.

In one embodiment of the method of the present invention, the preselected gene with therapeutic potential is a gene encoding a cytokine.

When none of the polypeptides selected in step a) shows a higher therapeutic index than the therapeutic index of reference, then none of said polypeptides are retained as suitable therapeutic agents.

According to a preferred embodiment of the invention, the determination of the therapeutic index of the polypeptides selected in step a) is achieved by means of the following steps:
i) submitting the polypeptides selected in step a) to at least two activity tests;
ii) attributing a value to each polypeptide in direct relation with the results of said activity tests; and
iii) determining the therapeutic index of each polypeptide from the values attributed in step ii).

In a preferred embodiment, the determination of the therapeutic index of the polypeptides selected in step a) is determined without resorting to *in vivo* activity tests.

The above step ii) can be performed by first, arbitrarily or not, attributing a preliminary value to the results of each activity test, which means that a relative value is given to the results of each activity test, this value being established by comparison with the results obtained for a reference product. Step iii) can be performed by integrating said preliminary values attributed to the activity tests for each polypeptide, in a single significant value that reflects the suitability of said polypeptide for a given therapeutic application.

The determination of the therapeutic index may be done by applying coefficients to said preliminary values. In this case, the choice of the coefficients depends on the specifications sought by the experimenter considering a given therapeutic application. For example, one may consider that the results of a specific *in vitro* test should receive a lesser coefficient than the results of a relevant *in vivo* test.

In another embodiment of the invention, at least one activity test performed to determine the therapeutic index of the polypeptides selected in step a) may be carried out by means of a gene expression vector carrying a polynucleotide which encodes one of said polypeptides.

Such vectors may be of particular interest in the case where *in vivo* or *in situ* gene expression is sought, for example when the polypeptide is produced within a cell, and/or for gene therapy.

The polypeptides, which are retained as therapeutic agents according to the method of the present invention, may have an identical, similar or different pharmacological profile in comparison with that of the reference product used to determine said therapeutic index of reference.

Thus, the method of the invention may also provide new therapeutic agents having a pharmacological profile different from the pharmacological profile of the reference product.

By pharmacological profile is meant here the pharmacological effect(s) of a therapeutic agent.

Consequently, the method of the invention may provide new therapeutic agents suitable for therapeutic applications that are different from the therapeutic applications of the reference product.

The amino acid sequences of the polypeptides selected in step a) may be very similar one from each other. In one embodiment of the invention, the sequences of the mature form of all the polypeptides selected in step a) differ one from each other by less than 20 amino acids, preferably by less than 10 amino acids, more preferably by only one single amino acid.

The polypeptides carried out in the method of the present invention are preferably used in their mature form. The mature form is understood as the active form of the polypeptide.

Another aspect of the present invention includes a gene expression vector comprising a polynucleotide encoding a polypeptide obtainable according to the method of the present invention.

Such a gene expression vector can be used as a therapeutic agent, in particular for gene therapy.

A gene expression vector according to the present invention comprises a vector that carries said polynucleotide and that can be selected from among those known to those skilled in the art as being suitable for gene therapy. Such vectors can comprise sequences of retrovirus, adenovirus, AAV virus, herpes virus, Poxvirus, synthetic vectors, nude DNA, liposomes, biolistic, etc.

The preferred method for administering a gene expression vector according to the present invention to a patient in need thereof can be readily determined by one skilled in the art taking into account the nature of the disease or disorder to be treated.

According to another aspect, the invention provides therapeutic agents consisting of polypeptides, which are identified by the method described above.

According to still another aspect, the invention provides as a therapeutic agent, a polynucleotide encoding a therapeutic agent identified according to the method of the invention, said therapeutic agent consisting of a polypeptide, as well as a gene expression vector comprising said polynucleotide and/or a host cell comprising said gene expression vector.

Still another aspect of the present invention is to provide a therapeutic agent comprising:
- a derivative of a polypeptide identified according to the method of the present invention, said derivative preferentially resulting from drug optimisation technologies such as PEGylation, glycosylation, succinylation, which aim to further increase the therapeutic index of said polypeptide, and/or;
- a derivative of a polynucleotide encoding a polypeptide identified according to the method of the present invention, said derivative preferentially resulting from site-directed mutagenesis or directed evolution technologies devoted to further increasing therapeutic index of the polypeptide encoded by said polynucleotide.

Optimisation technologies such as PEGylation and glycosylation are known to those skilled in the art and are described, for example, in US patents N°5,382,657 and N°6,340,242, respectively. These technologies often act to increase the half-life and stability of the polypeptides and can be used to so treat the polypeptides identified by the methods of the present invention.

Still yet another aspect of the present invention is to provide a therapeutic agent comprising a recombinant polypeptide whose amino acid sequence comprises several, preferably all the natural genetic variations characterizing the polypeptides which may be obtained according to the methods of the present invention.

Indeed, when a polypeptide is retained as a therapeutic agent in step c) of the present invention, this can be correlated to at least one genetic variation in the amino acid sequence of said polypeptide, which distinguishes said polypeptide from the polypeptide encoded by another natural allelic variant of the preselected gene or of the related gene thereof. Said natural genetic variation(s) is (are) regarded as characterizing a polypeptide. Different natural genetic variations can be found in different polypeptides retained as therapeutic agents according to the method of the invention.

All these natural genetic variations could be advantageously combined in one single polypeptide by using recombination based technologies and techniques well known to those skilled in the art. It is expected thereby that such a recombinant polypeptide would combine all the benefits of each individual polypeptide and be useful as a therapeutic agent for one or possibly several therapeutic applications.

In a preferred embodiment of the present invention, said recombinant polypeptide may combine all the natural genetic variations associated with the polypeptides which were selected in step d) as having the highest or second highest therapeutic index either for one or for several therapeutic applications.

According to another embodiment of the present invention, one or more polypeptides selected as therapeutic agents by the method of the present invention may be used in combination for manufacturing a medicine or medicament, in order to combine the benefits of each individual polypeptide.

More generally, the therapeutic agents described above can be used, either individually or in combination, for the manufacture of a medicine for the treatment of a patient in need thereof. Said therapeutic agents are preferably provided in a therapeutically effective amount that can be determined by one skilled in the art.

Said medicine usually comprises at least one pharmaceutically acceptable excipient such as talc, arabic gum, lactose, starch, dextrose, glycerol, ethanol, magnesium stearate, cocoa butter, aqueous or non-aqueous vehicles, fatty substances of animal or vegetable origin, paraffinic derivatives, glycols, various wetting agents, dispersants or emulsifiers, and/or preservatives.

The therapeutic agents can be employed alone or in combination with other therapeutic compounds like cytokines, such as interleukins or interferons, for instance.

Formulations of the pharmaceutical compositions are advantageously adapted according to the mode of administration.

The pharmaceutical compositions can be administered by different routes of administration, including for example subcutaneously, percutaneously, intramuscularly, intravenously, orally, intranasally, vaginally, rectally, etc, all of which are well known to those skilled in the art.

Another aspect of the present invention involves the use of a therapeutically effective amount of a therapeutic agent obtainable by the method of the present invention, or a gene expression vector according to the present invention, for the manufacture of a medicine for the treatment of a patient in need thereof.

Yet still another aspect of the present invention concerns a method for treating a patient having genetic deficiencies comprising administering a therapeutically effective amount of the therapeutic agent previously defined, and a pharmaceutically acceptable carrier.

### EXPERIMENTAL SECTION

The present invention is illustrated by tests performed on the polypeptides encoded by natural allelic variants of three genes belonging to the interferon alpha gene family, designated hereafter as "IFNα": C122S IFNα-5, G45R IFNα-17, Q114H and V127D IFNα-21, K179E IFNα-21. The natural allelic variants of IFNα encoding these polypeptides have been identified and isolated as described in the inventor's patent applications No. PCT/EP02/05458, PCT/EP02/05229, PCT/EP02/04082, fully incorporated herein by reference. The polypeptides encoded by the natural allelic variants of IFNα are subjected to several activity tests, which permits evaluation of their suitability for given therapeutic applications. For each activity test, the results obtained with these polypeptides are also compared with those obtained with a reference product consisting of the polypeptide encoded by the wild-type allelic variant of IFNα-2 (IFNα-2b) chosen as representative of the interferon molecule used on the market. This product is available under the trademark Intron A® from Schering Plough.

### Example 1: Antiproliferative activity test on human lymphoblasts of Daudi Burkitt's cell line

In order to study the anti-proliferative effects of C122S IFNα-5, G45R IFNα-17, Q114H and V127D IFNα-21, K179E IFNα-21, and compare them with those of the reference product (wild-type IFNα-2), cells from human Daudi Burkitt's lymphoma cell line, hereinafter called "Daudi cells", are cultivated in the presence of concentrations of one of said polypeptides ranging from 0.001 to 10 ng/ml.

The results of the anti-proliferative activity measurements obtained for each polypeptide tested enabled calculation of the concentration of interferon inhibiting the cell proliferation by 50% (lC50 value). The lC50 values determined for each interferon are reported in Table I.

**Table I**

| **Interferon** | **IC50 (ng/ml)** |
|---|---|
| C122S IFNα-5 | 0.390 |
| G45R IFNα-17 | 0.006 |
| Q114HN127D IFNα-21 | 0.413 |
| K179E IFNα-21 | 0.024 |
| Wild-type IFNα-2 | 0.048 |

These results clearly demonstrate that the interferons tested have an antiproliferative activity on Daudi cells. Furthermore, the G45R IFNα-17 has the highest capacity to inhibit Daudi cells proliferation, which is also higher than that of the wild-type IFNα-2. The K179E IFNα-21 also has a higher capacity to inhibit Daudi cells proliferation than that of the wild-type IFNα-2.

### Example 2: Antiviral activity tests

The IFNs play an important role in the antiviral defence in mammals. The IFN antiviral activity is partly due to IFN induced enzymatic systems, such as:
- The 2',5'-oligoadenylate synthetase, an enzyme which catalyzes the adenosine oligomer synthesis. These oligomers activate the RNase L, an endoribonuclease which destroys the viral RNA once activated.
- The Mx proteins (GTPases) which inhibit the synthesis and/or the maturation of viral transcripts. This activity is mainly exerted on the influenza virus.
- The PKR protein (or p68 kinase) which is activated by the double-stranded RNA. The activated PKR inhibits viral protein synthesis.

The IFNs antiviral activity is also induced by other mechanisms such as, in the case of retroviruses, the inhibition of viral particle entry into the cells, the replication, the binding, the exit of the particles and the infective power of viral particles.

Finally, the IFNs exert an indirect antiviral activity by modulating certain functions of the immune system, in particular by favoring the response to cellular mediation (including an increase in the expression of the MHC class I and II molecules, increase in IFN-gamma production, increase in the CTL activities, among others).

The antiviral activity of C122S IFNα-5, G45R IFNα-17, Q114H and V127D IFNα-21, and K179E IFNα-21 has been evaluated both in cell culture and in mouse model. Both tests have been carried out in parallel with wild-type IFNα-2 used as the reference product.

### a) Antiviral activity in cell culture

This assay permits evaluation of the antiviral activity of C122S IFNα-5, G45R IFNα-17, Q114H and V127D IFNα-21, and K179E IFNα-21 in cell culture using the vesicular stomatitis virus (VSV), and comparison with the anti-viral activity of the reference product (wild-type IFNα-2).

To do so, WISH human epithelial cells were cultivated for 24 hours in the presence of decreasing concentrations of each interferon. Thereafter the cells were infected by the vesicular stomatitis virus (VSV) for 24 to 48 additional hours and cell lysis measured.

The antiviral effect of the different IFNα tested was determined by comparing the IC50 value corresponding to the IFN concentration inhibiting 50% of cell lysis induced by the VSV.

A similar experiment was carried out three times, and the IC50 values measured in one representative experiment are presented in Table II.

**Table II**

| **IFNα polypeptides** | **IC50 (ng/ml)** |
|---|---|
| C122S IFNα-5 | 17 |
| G45R IFNα-17 | 2 |
| Q114H/V127D IFNα-21 | 22 |
| K179E IFNα-21 | 25 |
| Wild-type IFNα-2 | 4 |

The results of this experimentation indicate that the interferons tested exhibited antiviral activity in cell culture. In particular, among the interferons tested, the G45R IFNα-17 possesses the highest antiviral activity in cell culture infected with VSV, which is also higher than that of wild-type IFNα-2.

### b) Antiviral activity in mouse model

This test is performed in the EMCV (Encephalomyocarditis virus) mouse model.

Human IFNs exhibit dose-dependent antiviral activity in the mouse which is in general 100 to 1,000 fold less than that exhibited by the same amount of mouse IFN (Meister et al. (1986), J. Gen. Virol. 67, 1633-1644).

Intraperitoneal injection of mice with Encephalomyocarditis virus (EMCV) gives rise to a rapidly progressive fatal disease characterized by deleterious effects on the central nervous system and encephalitis (Finter NB (1973), Front Biol. 2: 295-360). Mouse and human interferon-alpha have both been shown to be effective in protecting mice against lethal EMCV infection (Tovey and Maury (1999), J. IFN Cytokine Res. 19: 145-155).

Groups of 20, six-week old Swiss mice were infected intraperitoneally with 100 x LD₅₀ EMCV and treated one hour later, and then once daily for 3 days thereafter with 2 µg of each interferon. A negative control group was performed with animals having been treated with excipient only. The animals were followed daily for survival for 21 days.

Results are presented in Figure 1 and indicate that the relative survival rate of the mice which had been treated with interferons is much higher than the survival rate of the non-treated mice, demonstrating the antiviral activity, in mouse model, of the interferons tested. The antiviral activity exhibited by the G45R IFNα-17 and Q114H and V127D IFNα-21 in mouse model was higher than that observed for the mice which have been treated with wild-type IFNα-2. Moreover, among all the interferons tested, the G45R IFNα-17 exhibits the highest antiviral activity in mouse model.

### Example 3: Immunomodulatory activity tests

IFNs type I (IFN alpha and IFN beta) are able to modulate certain functions of the immune system. The immunomodulatory activity of the interferons can be evaluated in parallel on dendritic cell maturation and in mice previously inoculated with malignant Friend erythroleukemia cells.

### a) Effect on dendritic cell maturation.

Immunomodulatory activity of C122S IFNα-5, G45R IFNα-17, Q114H and V127D IFNα-21, and K179E IFNα-21 was first investigated on dendritic cell maturation and compared with that of the reference product (wild-type IFNα-2).

To do so, dendritic cells were first generated from adult human peripheral blood monocytes cultivated in the presence of GM-CSF and IL-4 cytokines. After purification using a CD14+ cells purification kit, these dendritic cells were placed in the presence of 100 ng/ml of each interferon, and their phenotype determined by FACS analysis. The analysis was focused upon determination of expression of the MHC class I and II molecules and the CD40, CD80, CD86, CD83 and CD1a cell membrane markers. The maturation state of these dendritic cells was also compared with that obtained without IFNα (no IFNα) treatment to provide a negative control with non-stimulated dendritic cells, and with treatment with either TNF-α (Tumor Necrosis Factor-α, 100 ng/ml) or LPS (Lipopolysaccharide, 1 µg/ml), to provide positive controls.

The median value of the measurements of fluorescence intensity (FACS) for each marker, expressed as arbitrary units, are presented in Table III.

**Table III**

| | **HLA ABC** | **HLA DR** | **CD40** | **CD80** | **CD86** | **CD83** | **CD1a** |
|---|---|---|---|---|---|---|---|
| No IFNα | 64 | 133 | 24 | 25 | 14 | 15 | 26 |
| LPS | 188 | 325 | 567 | 151 | 67 | 17 | 126 |
| TNFα | 72 | 209 | 355 | 49 | 9 | 13 | 181 |
| C122S IFNα-5 | 136 | 217 | 621 | 132 | 58 | 16 | 113 |
| Q114H V127D IFNα-21 | 68 | 122 | 163 | 46 | 8 | 8 | 191 |
| G45R IFNα-17 | 80 | 192 | 213 | 44 | 25 | 11 | 149 |
| K179E IFNα-21 | 181 | 322 | 491 | 87 | 44 | 14 | 127 |
| Wild-type IFNα-2 | 87 | 281 | 331 | 76 | 45 | 15 | 155 |

The results of this test demonstrate that the capacity to stimulate dendritic cell maturation varies according to the interferon tested. In particular, the C122S IFNα-5 and the K179E IFNα-21 exhibit the highest capacity to stimulate dendritic cell maturation, the activity of both polypeptides is higher than that of wild-type IFNα-2.

### b) Effect on survival of mice previously inoculated with malignant Friend erythroleukemia cells

IFNα has been shown to be equally effective in protecting mice against the growth of a clone of Friend leukemia cells resistant to the direct anti-proliferative activity of IFNα *in vitro* as against IFN sensitive parental Friend leukemia cells (Belardelli et al., Int. J. Cancer, 30, 813-820, 1982; Belardelli et al., Int. J. Cancer, 30, 821-825, 1982), reflecting the importance of indirect immune mediated mechanisms in the anti-tumoral activity of IFNα.

The following experimentation permitted evaluation of the anti-tumoral activity of C122S IFNα-5, G45R IFNα-17, Q114H and V127D IFNα-21, and K179E IFNα-21 in mice previously inoculated with Friend erythroleukemia cells, and comparison with that of the reference product (wild-type IFNα-2).

To perform the experiments, groups of 12 six-week old DBA/2 mice were inoculated intraperitoneally with 100,000 IFN resistant Friend leukaemia cells (3C18) (20,000 LD₅₀) and treated one hour later and then once daily for 21 days thereafter with 2.0 µg of each interferon or an equivalent volume of excipient alone. The animals were then followed daily for survival, for 70 days.

The results of this experiment, presented in Figure 2, indicate that, compared with mice which have not been treated with IFNα, treatment of mice with any of C122S IFNα-5, G45R IFNα-17, Q114H and V127D IFNα-21, and K179E IFNα-21 results in an increase in the number of mice surviving 70 days after inoculation with highly malignant Friend erythroleukemia cells (FLC). In particular, the survival of FLC inoculated mice was the highest after treatment with K179E IFNα-21 and was also very high with Q114H and V127D IFNα-21. The survival rate measured after treatment with one of these two polypeptides was higher than after treatment with wild-type IFNα-2.

### Example 4: Toxicity tests

Like most other cytokines, the interferons alpha are produced to act locally in the body. Therefore, their systemic pharmacological use induces toxic effects. The most consistent acute effect reported during interferon alpha therapy, after each administration of interferon, in a majority of patients is the "flu syndrome" that results in fever, fatigue, weight and appetite loss. This syndrome has also been reported, after each administration, in Rhesus monkeys which are considered to be very close to humans. Moreover, cardiologic effects have also been reported as adverse effects caused by interferons alpha administered in systemic.

Therefore, safety pharmacological study was performed in conscious Rhesus monkeys monitored by telemetry to test the effect on temperature, arterial blood pressure, and heart rate, of an acute subcutaneous administration of each interferon.

In this model, for each animal, body temperature and haemodynamic parameters were recorded 2 hours before the administration of interferon and during 24 hours following the administration. For each of the interferons tested, the doses of interferon administered subcutaneously were 1 and 10 µg/kg in two animals and 20 µg/kg in one animal. A negative control corresponding to administration of excipient only was also performed demonstrating that the administration of vehicle had no effect on body temperature (the first clinical sign usually observed), arterial blood pressure, or heart rate.

As indicated in figure 3, the subcutaneous administration of 1 µg/kg, 10 µg/kg or 20 µg/kg of C122S IFNα-5 had no significant effect on body temperature in monkeys. As shown in figure 3, among the five interferons tested, the C122S IFNα-5 exhibited the lowest toxicity that was estimated by the increase in body temperature of the monkey. Compared with the other interferons tested, the K179E IFNα-21 exhibited a low toxicity too. Both the C122S IFNα-5 and K179E IFNα-21 exhibit a lower toxicity in monkeys than the wild-type IFNα-2.

Similarly, at all tested doses, the administration of any of the interferons tested had no significant effect on arterial blood pressure and heart rate in monkeys.

### Example 5: Therapeutic index determination

The therapeutic index of each polypeptide tested in examples 1 to 4 was determined on the basis of the results obtained from the activity tests described in those examples.

In fact, for each polypeptide tested, three different therapeutic indexes with regard to the three investigated therapeutic applications were determined, as follows:
- an antiviral therapeutic index related to the antiviral application was determined using the average value calculated for the antiviral activity and the average value calculated for the toxicity;
- an antiproliferative therapeutic index related to the antiproliferative application was determined using the average value calculated for the antiproliferative activity and the average value calculated for the toxicity; and
- an immunomodulatory therapeutic index related to the immunomodulatory application was determined using the average value calculated for the immunomodulatory activity and the average value calculated for the toxicity.

Therefore, each therapeutic index reflects the suitability of a tested polypeptide in a given therapeutic application.

More particularly, in order to determine said therapeutic index, one can attribute a preliminary value, advantageously comprised between -3 and +3, to each result of the activity tests performed for each polypeptide.

These preliminary values were attributed arbitrarily by the experimenter in the following manner:
When the result obtained for a polypeptide in one activity test was equal or close to the one obtained for the reference product (the wild-type IFNα-2), the attributed preliminary value was 0. When the result was regarded as slightly, strongly or very strongly above the result of the reference product, the attributed preliminary value was +1, +2 and +3, respectively. Similarly, when the result was slightly, strongly or very strongly below the result of the reference product, the attributed preliminary value was -1, -2 and -3, respectively. As the preliminary values for the polypeptides tested were determined in comparison with the result obtained with the reference product, the preliminary values for the reference product are all necessarily equal to 0.

Furthermore, the experimenter arbitrarily applied coefficients to each activity test, said coefficients were applied to the preliminary values as determined above, in order to calculate the average value for the corresponding activity.

Thus, for each activity (such as antiviral activity, antiproliferative activity, immunomodulatory activity, and toxicity, in this precise case), one can determine an average value by adding the preliminary values of each activity test, modified by their respective coefficients. The results so obtained are described in Table IV presented below.

Afterwards, for each polypeptide tested, the therapeutic index was determined by subtracting the toxicity average value, which was furthermore modifed by a coefficient of 2/3, to the sum of the average values obtained for the activities which are relevant to the intended therapeutic application.

The coefficients applied to each of the preliminary values of the activity tests for the calculation of the average value depend upon the relevance of a given activity test relative to another with regard to a given therapeutic application. The coefficients applied to each of the average values of the activities for the calculation of the therapeutic index depend upon the relevance of a given activity relative to another with regard to a given therapeutic application.

For example, a coefficient of 0.4 was applied to the antiviral activity test performed in cell culture, while a coefficient of 0.6 was applied to the antiviral activity test performed in mice. This was done because with regard to the antiviral application, it was determined that the test performed in cell culture was less relevant than the one performed in mice.

Since the therapeutic index of reference is determined from the preliminary values obtained for the reference product, which are all equal to 0, therefore, in the present case, the therapeutic index of reference is equal to 0.

The therapeutic indexes of the tested interferons and of the reference product obtained as described above are shown in table V hereafter.

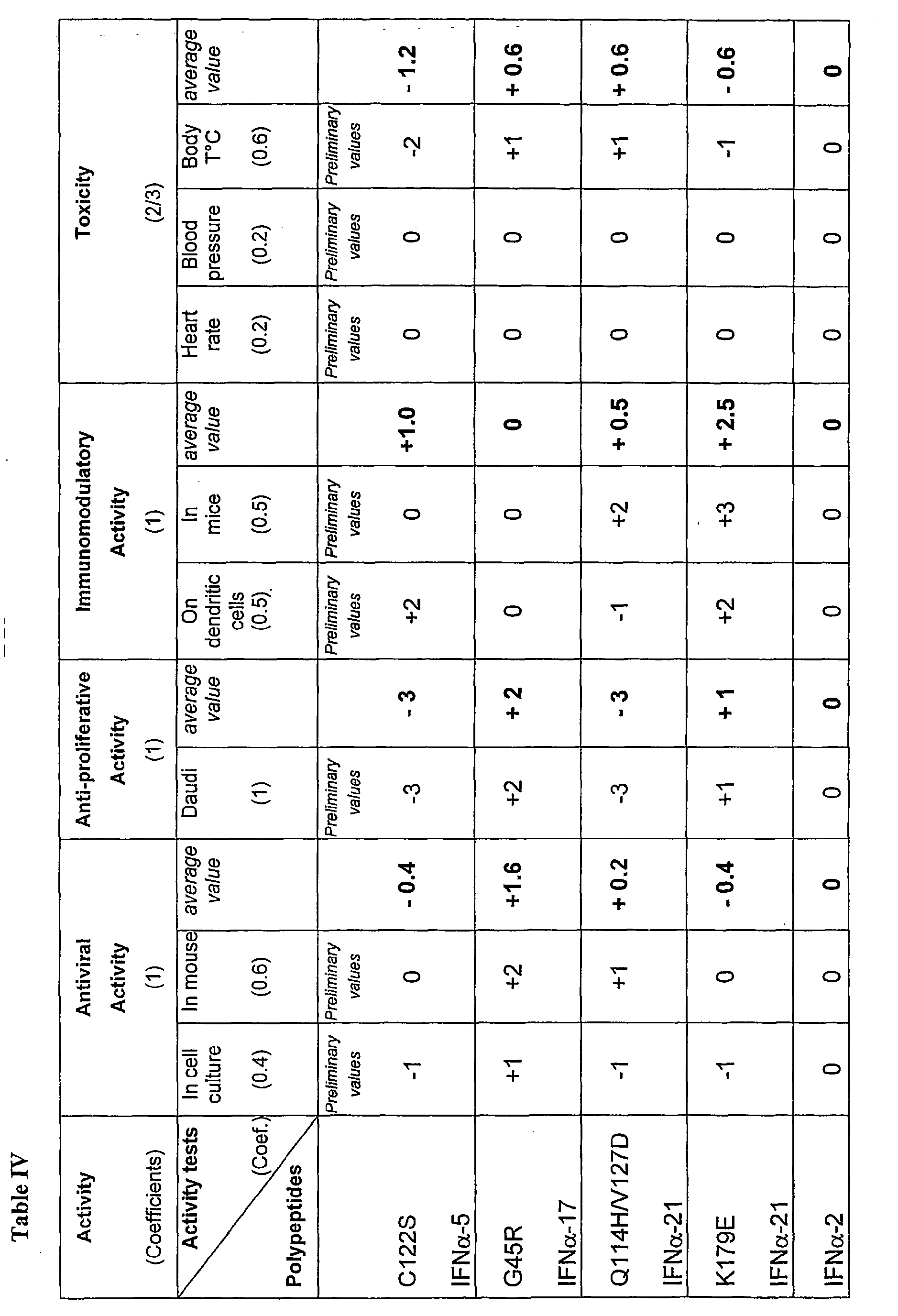

**Table V**

| | **Therapeutic index** | | |
|---|---|---|---|
| | **Antiviral application** | **Antiproliferative application** | **Immunomodulatory application** |
| | *values* | *values* | *values* |
| C122S | +0.4 | -2.2 | +1.8 |
| IFNα-5 | | | |
| G45R | **+1.2** | **+1.6** | **-0.4** |
| IFNα-17 | | | |
| Q114H/V127D | -0.2 | -3.4 | +0.1 |
| IFNα-21 | | | |
| K179E | 0 | **+ 1.4** | **+ 2.9** |
| IFNα-21 | | | |
| IFNα-2 | 0 | 0 | 0 |
| (reference product) | (index of reference) | (index of reference) | (index of reference) |

The results so obtained demonstrate, for the first time, a disassociation between the different kinds of activities of a pleiotropic polypeptide. This means that each one of these activities may be differently affected by a natural genetic variation, i.e. the polypeptide encoded by a natural allelic variant of the interferon may have a higher immunomodulatory activity, for example, but a lesser or similar toxicity in comparison with the wild-type interferon on the market. As a consequence, the method of the present invention can provide new therapeutic agents with new pharmacological profiles.

These results also demonstrate that the natural allelic variants of a preselected gene with therapeutic potential and the natural allelic variants of genes belonging to the same gene family constitute an actual source of molecules which can be efficiently used as therapeutic agents. Indeed, these results indicate that:
- the therapeutic index of G45R IFNα-17 and the therapeutic index of C122S IFNα-5 are higher than that of wild-type IFNα-2 in therapeutic applications where antiviral activity is required. In particular G45R IFNα-17 has the highest therapeutic index for said therapeutic applications. Thus, C122S IFNα-5 and, still more preferably, G45R IFNα-17, or any other polynucleotide encoding said polypeptides, may be used as therapeutic agents to treat diseases or disorders requiring an antiviral activity;
- the therapeutic index of G45R IFNα-17 and the therapeutic index of K179E IFNα-21 are higher than that of the reference product and are the highest and second highest, respectively, in therapeutic applications where antiproliferative activity is required. Thus, one of these two polypeptides or both, or any polynucleotide encoding said polypeptides, may be used as therapeutic agents to treat diseases or disorders requiring an antiproliferative activity; and
- the therapeutic index of K179E IFNα-21 and the therapeutic index of C122S IFNα-5 are higher than that of the reference product and are the highest and second highest, respectively, in therapeutic applications where immunostimulatory activity is required. Thus, K179E IFNα-21 and/or C122S IFNα-5, or any polynucleotide encoding said polypeptides, may be used as therapeutic agents to treat diseases or disorders requiring an immunostimulatory activity.

In addition, the method of the present invention provides new therapeutic agents suitable for new therapeutic applications. Indeed, the results disclosed in these examples demonstrate that, in contrast to the reference product which is known to be used to treat melanoma (requiring antiproliferative activity) and hepatitis C (requiring antiviral activity) but not to treat diseases or disorders requiring immunomodulatory activity, some therapeutic agents selected with the method of the invention i.e. the K179E IFNα-21 and C122S IFNα-5 polypeptides have a higher therapeutic index than the reference product in therapeutic applications where immunostimulatory activity is required.

All references cited herein are fully incorporated by reference.

It is to be understood that the examples provided herein are provided for explanatory purposes and are not to be construed as limited and that numerous variations may be made including choice of target polypeptides without departing from either the spirit or scope of the present invention.

## Claims

1. A method for identifying new therapeutic agent(s), **characterized in that** it comprises the following steps:
a) providing a reference product having a therapeutic index, said reference product being known to be useful in a least one therapeutic application and said therapeutic index corresponding to a balance between beneficial and adverse effects of said reference product;
b) selecting at least one polypeptide encoded by a natural allelic variant of one preselected gene with therapeutic potential belonging to the gene families of cytokines;
c) determining the therapeutic index of the polypeptide(s) selected in step b), said therapeutic index corresponding to a balance between beneficial and adverse effects of said polypeptide(s) selected in step b), said adverse effects not being determined through *in vivo* toxicity tests performed in humans;
d) retaining as therapeutic agent(s), the polypeptide(s) selected in step b) whose therapeutic index, as determined in step c), is higher than the therapeutic index of the reference product.

2. The method according to claim 1, wherein the therapeutic index in step c) is determined without resorting to *in vivo* activity tests.

3. The method according to claim 1 or 2, **characterized in that** at least 2 polypeptides, preferably at least 4 polypeptides, are selected in step b).

4. The method according to any one of claims 1 to 3, **characterized in that** it further comprises step e), wherein only the polypeptide(s) retained in step d) which has (have) the highest or second highest therapeutic index, is (are) selected as therapeutic agent(s).

5. The method according to any one of claims 1 to 4, wherein said natural allelic variants originate from the same species.

6. The method according to claim 5, wherein said natural allelic variants originate from the human species.

7. The method according to any one of claims 1 to 6, wherein the polypeptides selected in step b) are under their mature form.

8. The method according to any one of claims 1 to 7, wherein the amino acid sequences of the mature form of all the polypeptides selected in step b) do not differ one from each other by more than 20 amino acids, preferably by more than 10 amino acids, more preferably by only one single amino acid.

9. The method according to any one of claims 1 to 8, wherein at least one activity test is carried out by means of a gene expression vector carrying a polynucleotide which encodes one of the polypeptides selected in step b).

10. The method according to any one of claims 1 to 9, wherein the therapeutic index in step a) is determined from data found in the scientific literature.

## Patentansprüche

1. Verfahren zur Identifizierung eines neuen therapeutischen Mittels/neuer therapeutischer Mittel, **dadurch gekennzeichnet, dass** es die folgenden Schritte umfasst:
a) Bereitstellen eines Referenzprodukts, das einen therapeutischen Index hat, wobei von dem Referenzprodukt bekannt ist, dass es in wenigstens einer therapeutischen Anwendung nützlich ist, und der therapeutische Index einem Gleichgewicht zwischen günstigen Wirkungen und nachteiligen Wirkungen des Referenzprodukts entspricht;
b) Auswählen wenigstens eines Polypeptids, das durch eine natürliche allelische Variante eines vorher ausgewählten Gens mit therapeutischem Potential, das zu der Gen-Familie der Cytokine gehört, codiert wird;
c) Bestimmen des therapeutischen Index des Polypeptids/der Polypeptide, ausgewählt in Schritt b), wobei der therapeutische Index einem Gleichgewicht zwischen günstigen Wirkungen und nachteiligen Wirkungen des Polypeptids/der Polypeptide, ausgewählt in Schritt b), entspricht, wobei die nachteiligen Wirkungen durch *in vivo-*Toxizitätstests, die beim Menschen durchgeführt werden, nicht bestimmt werden;
d) Zurückbehalten als therapeutisches Mittel/therapeutische Mittel das Polypeptid/die Polypeptide, ausgewählt in Schritt b), dessen/deren therapeutischer Index, wie er in Schritt c) bestimmt wird, höher ist als der therapeutische Index des Referenzprodukts.

2. Verfahren gemäß Anspruch 1, wobei der therapeutische Index in Schritt c) bestimmt wird, ohne dass auf *in vivo*-Aktivitätstests zurückgegriffen wird.

3. Verfahren gemäß Anspruch 1 oder 2, **dadurch gekennzeichnet, dass** wenigstens zwei Polypeptide, vorzugsweise wenigstens vier Polypeptide, in Schritt b) ausgewählt werden.

4. Verfahren gemäß einem der Ansprüche 1 bis 3, **dadurch gekennzeichnet, dass** es außerdem Schritt e) umfasst, in dem nur das Polypeptid/die Polypeptide, zurückbehalten in Schritt d), das/die den höchsten oder zweithöchsten therapeutischen Index hat/haben, als therapeutisches Mittel/therapeutische Mittel ausgewählt wird/werden.

5. Verfahren gemäß einem der Ansprüche 1 bis 4, wobei die natürlichen allelischen Varianten von derselben Spezies stammen.

6. Verfahren gemäß Anspruch 5, wobei die natürlichen allelischen Varianten von der Spezies Mensch stammen.

7. Verfahren gemäß einem der Ansprüche 1 bis 6, wobei die in Schritt b) ausgewählten Polypeptide in ihrer reifen Form sind.

8. Verfahren gemäß einem der Ansprüche 1 bis 7, wobei die Aminosäure-Sequenzen der reifen Form aller Polypeptide, ausgewählt in Schritt b), sich nicht durch mehr als 20 Aminosäuren, vorzugsweise nicht durch mehr als 10 Aminosäuren, bevorzugter nur durch eine einzelne Aminosäure voneinander unterscheiden.

9. Verfahren gemäß einem der Ansprüche 1 bis 8, wobei wenigstens ein Aktivitätstest mittels eines Gen-Expressionsvektors, der ein Polynucleotid trägt, das eines der in Schritt b) ausgewählten Polypeptide codiert, durchgeführt wird.

10. Verfahren gemäß einem der Ansprüche 1 bis 9, wobei der therapeutische Index in Schritt a) aus Daten bestimmt wird, die in der wissenschaftlichen Literatur gefunden werden.

## Revendications

1. Procédé d'identification d'un nouvel agent thérapeutique/de nouveaux agents thérapeutiques, **caractérisé en ce qu'**il comprend les étapes suivantes:
a) fournir un produit de référence ayant un index thérapeutique connu, ledit produit de référence étant utile dans au moins une utilisation thérapeutique, et ledit index thérapeutique correspondant à un équilibre entre les effets bénéfiques et les effets indésirables dudit produit de référence;
b) sélectionner au moins un polypeptide, encodé par un variant allélique naturel d'un gène présélectionné appartenant à la famille des cytokines ayant un potentiel thérapeutique;
c) déterminer l'index thérapeutique du ou des polypeptides sélectionnés à l'étape b), ledit index thérapeutique correspondant à un équilibre entre les effets bénéfiques et les effets indésirables du ou des polypeptides sélectionnés à l'étape b), lesdits effets indésirables n'étant pas déterminés par des tests de toxicité *in vivo* chez l'homme;
d) retenir comme agent(s) thérapeutique(s) les polypeptide(s) sélectionné(s) à l'étape b), celui (ceux) dont l'index thérapeutique, tel que déterminé à l'étape c), est supérieur à l'index thérapeutique du produit de référence.

2. Procédé selon la revendication 1, dans lequel l'index thérapeutique de l'étape c) est déterminé sans recourir à des tests d'activité *in vivo.*

3. Procédé selon la revendication 1 ou 2, **caractérisé en ce qu'**au moins 2 polypeptides, de préférence au moins 4 polypeptides, sont sélectionnés à l'étape b).

4. Procédé selon l'une quelconque des revendications 1 à 3, **caractérisé en ce qu'**il comprend également l'étape e), dans laquelle seul(s) le(s) polypeptides(s) retenu(s) à l'étape d), ayant l'index thérapeutique le plus élevé ou le deuxième index le plus élevé, est (sont) sélectionné(s) en tant qu'agent(s) thérapeutique(s).

5. Procédé selon l'une quelconque des revendications 1 à 4, dans lequel lesdits variants alléliques naturels proviennent de la même espèce.

6. Procédé selon la revendication 5, dans lequel lesdits variants alléliques naturels proviennent de l'espèce humaine.

7. Procédé selon l'une quelconque des revendications 1 à 6, dans lequel les polypeptides sélectionnés à l'étape b) sont sous une forme mature.

8. Procédé selon l'une quelconque des revendications 1 à 7, dans lequel les séquences d'acides aminés de la forme mature de l'ensemble des polypeptides sélectionnés à l'étape b) ne diffèrent pas les unes des autres par plus de 20 acides aminés, de préférence par plus de 10 acides aminés, et plus préférentiellement, diffèrent par seulement un seul acide aminé.

9. Procédé selon l'une quelconque des revendications 1 à 8, dans lequel au moins un test d'activité est réalisé au moyen d'un vecteur d'expression porteur d'un polynucléotide encodant l'un des polypeptides sélectionnés à l'étape b).

10. Procédé selon l'une quelconque des revendications 1 à 9, dans lequel l'index thérapeutique de l'étape a) est déterminé à partir de données tirées de la littérature scientifique.
